# EUROPEAN PATENT APPLICATION

(11) **EP 2 404 500 A1**
(43) Date of publication of application: **11.01.2012**
(21) Application number: 10305758.4
(22) Date of filing: 07.07.2010
(51) Int. Cl.: A01K 67/027, C12N 15/85, A61K 49/00

(54) **A transgenic animal as a model for identifying adult stem cells, and uses thereof**

(71) Applicant: Université Pierre et Marie Curie - Paris 6, 75005 Paris (FR)
(72) Inventor: Sassoon, David, 75007 Paris (FR); Besson, Vanessa, 78160 Marly le Roi (FR); Marazzi, Giovanna, 75007 Paris (FR)
(74) Representative: Chajmowicz, Marion

(57) **Abstract**

The present invention relates to the use of a transgenic non-human animal, such as a mouse, expressing a reporter gene detectable by a chromogenic, luminescent or fluorescent signal which identifies the cells that express Pw1, or of Pw1-expressing cells or tissues isolated therefrom, as a model for screening a candidate substance for its ability to stimulate adult stem cells, or for monitoring cell aging.

## Description

The present invention relates to identification of adult stem cells in a transgenic non-human animal, which is useful as a tool for screening for pharmaceutical drugs that act upon the stem cells, and for monitoring cell aging.

### Background of the invention

Stem cells are defined by the ability to continuously self-renew and produce the differentiated progeny of the tissue of their location (Morrison et al., 1997). Stem cells are a small percentage of the total cells. For instance, in the small intestine there are perhaps up to 10 stem cells near the bottom of the crypt out of a total crypt population of <300 cells. In skeletal muscle, satellite (stem) cells comprise about 5% of all nuclei, but in the bone marrow the multi-potential hematopoietic stem cell is much rarer, with a frequency of perhaps 1 in 10000 amongst all bone marrow cells. Considerable overlap exists between different putative organ specific stem cells in their repertoire of gene expression, often related to self-renewal, cell survival and cell adhesion. More robust tests of 'stemness' are now being employed, using lineage-specific genetic marking and tracking to show production of long-lived *clones in vitro* and multi-potentiality *in vivo.* However, the conditions to grow or simply 'select' stem cells *in vitro* do not exist for many tissues where it is accepted that the stem cells simply fail to grow due to lack of required growth factors or substrates. In many tissues, the stem cells have simply not been identified.

Nevertheless stem cells have been used routinely for more than three decades to repair tissues and organs damaged by injury or disease, most notably from bone marrow. While early, embryonic stem cells have generated considerable interest, adult stem cells are critical for tissue homeostasis and wound repair and reside within specific niches that preserve proliferative and regenerative potential (Blanpain and Fuchs, 2006; Moore and Lemischka, 2006).

Understanding how stem cells are maintained, stimulated and participate in regeneration is important to a wide variety of diseases as well as potential targets in the aging population. These somatic, or adult, stem cells are undifferentiated cells that reside in differentiated tissues, and have the properties of self-renewal and generation of differentiated cell types. The differentiated cell types may include all or some of the specialized cells in the tissue. Sources of somatic stem cells include bone marrow, blood, the cornea and the retina of the eye, brain, skeletal muscle, dental pulp, liver, skin, the lining of the gastrointestinal tract, and pancreas. Adult stem cells are usually quite sparse. Often they are difficult to identify, isolate, and purify. As a result, stem cells must be identified prospectively and purified carefully in order to study their properties.

However, very few markers of adult stem cells have been identified so far. For instance colon cancer research has led to the identification of leucine-rich repeat-containing G-protein coupled receptors Lgr5 and Lgr6 that are expressed by adult stem cells ((Barker et al., 2010); international patent application WO2009/022907). Pw1/Peg3, that is a maternally imprinted gene expressed during skeletal myogenesis, has also been proposed as a stem cell marker. For instance, a population of muscle-resident stem cells was identified in the interstitium that expresses the cell stress mediator PW1 but not other markers of muscle stem cells such as Pax7. PW1(+)/Pax7(-) interstitial cells (PICs) are *myogenic in vitro* and efficiently contribute to skeletal muscle regeneration *in vivo* as well as generating satellite cells and PICs. Furthermore, it was found that PICs are not derived from a satellite cell lineage. Taken together, these findings uncover an anatomically identifiable population of muscle progenitors (Mitchell et al., 2010). To characterize the role of *Pw1* as a potential marker of multiple stem cell populations, a reporter mouse, called *Tg(Pw1^{IRES-nLacZ})* , was generated (Besson et al, abstract published in April 2008, New-Orleans (New directions in Biology and Disease of skeletal muscle).

There remains a need for methods for identifying and culturing adult stem cells, and for rapid and reliable identification or screening of substances that act on adult stem cells.

### Summary of the invention

A subject of the invention is the use of a transgenic non-human animal expressing a reporter gene detectable by a chromogenic, luminescent or fluorescent signal which identifies the cells that express PW1, or of PW1-expressing cells or tissues isolated therefrom, as a model for screening a candidate substance for its ability to stimulate adult stem cells.

More particularly the invention provides *an in vitro* method for screening a candidate substance for its ability to stimulate adult stem cells, which method comprises the steps consisting of:
a. Providing adult stem cells isolated from a transgenic non-human animal expressing a reporter gene detectable by a chromogenic, luminescent, or fluorescent signal which identifies the cells that express PW1 that is a marker of adult stem cells;
b. Contacting said stem cells with the candidate substance;
c. Determining the ability of the candidate substance to trigger, maintain or enhance the chromogenic, luminescent or fluorescent signal, whereby identifying the substance as being able to stimulate adult stem cells.

The invention further provides *an in vivo* method for screening a candidate substance for its ability to stimulate adult stem cells, which method comprises administering a transgenic non-human animal expressing a reporter gene detectable by a chromogenic, luminescent or fluorescent signal which identifies the cells that express PW1, that is a marker of adult stem cells, with the candidate substance; and determining the ability of the candidate substance to trigger, maintain or enhance the chromogenic, luminescent or fluorescent signal, whereby identifying the substance as being able to stimulate adult stem cells.

Another subject of the invention is a method for cultivating non-human animal adult stem cells, which method comprises the steps consisting of:
a. Providing a transgenic non-human animal expressing a reporter gene detectable by a chromogenic, luminescent or fluorescent signal which identifies the cells that express Pw1, that is a marker of adult stem cells;
b. Isolating adult stem cells from the transgenic non-human animal by analyzing the chromogenic, luminescent or fluorescent signal of the reporter gene, optionally coupled to an analysis of stem cell marker(s);
c. Allowing the isolated adult stem cells to *grow in vitro.*

Still another subject of the invention is the use of a transgenic non-human animal expressing a reporter gene detectable by a chromogenic, luminescent or fluorescent signal which identifies the cells that express PW1, or of PW1-expressing cells or tissues isolated therefrom, as a model for monitoring cell aging.

Preferably the non-human animal is a mammal, more preferably a rodent, still more preferably a mouse.

### Legends to the Figures

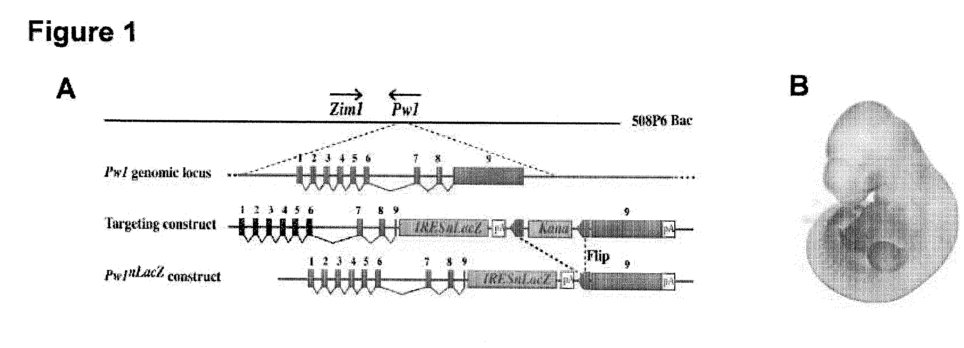

**Figure 1A** is a schematic drawing of the BAC recombination strategy. Reporter mice have been generated using BAC recombineering strategy by introducing an IRESnLacZ cassette in the 5'part of the BAC exon 9. The BAC has been randomly integrated in the genome by classical transgenesis methods known to those in the field. The wild-type *Pw1* locus with its open reading frame from exon 1 to 9 (black boxes) is located into the 508P6 BAC (180 kb). *Zim1* gene is located at less than 40 kb *from Pw1.* The arrows indicate the direction of gene transcription. An *IRESnLacZ-pA* cassette and a *kanamycin* (*Kana*) gene floxed by two FRT sites (triangles) were introduced into the 5' part of *Pw1* exon 9. The *Tg(Pw1^{IRES-nLacZ})* BAC construct was generated by FRT sites recombination and injected in mice to establish the reporter line.
**Figure 1B** is a photo that shows the reporter expression profile in an E10.5 mouse embryo using X-gal staining. This profile is identical to *Pw1* mRNA and PW1 protein expression (data not shown)
**Figures 2A and 2B** show photomicrographs of representative cross-sections of adult *Tg(pw1^{IRES-Lacz})* reporter mouse muscle, showing the expression of PW1 reporter in interstitial cells (PICs or PW1 interstitial cells) and Satellite Cells.

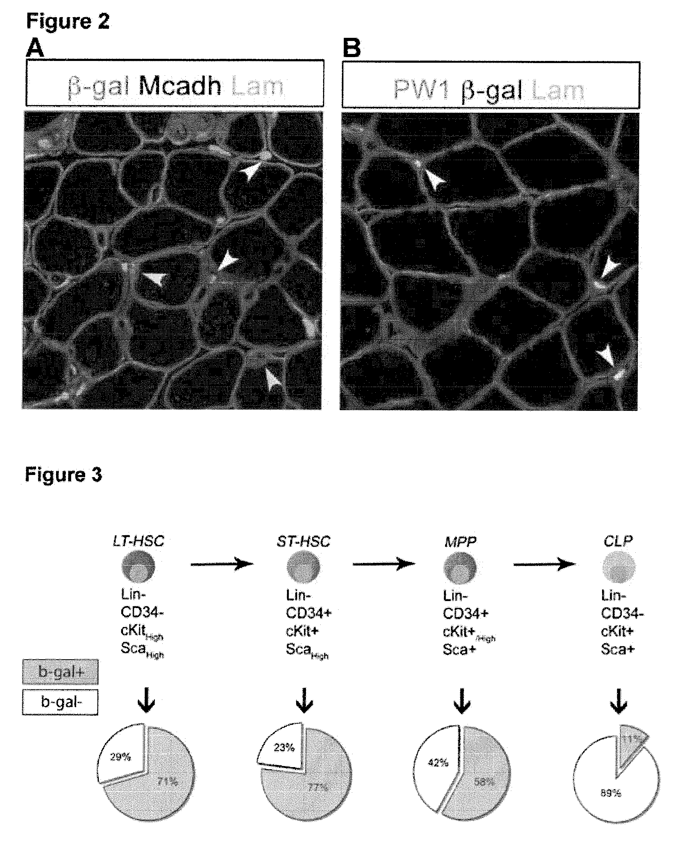

Figure 2A shows muscle cross-sections immunostained for ß-galactosidase (green) mcadherin (red), a satellite cell marker and laminin (orange), a basal lamina marker. PW1 reporter co-localizes with m-cadherin in satellite cells and marks a subset of interstitial cells.
Figure 2B shows muscle cross-sections immunostained for ß-galactosidase (red), Pw1 (green) and laminin (orange). PW1 reporter colocalizes perfectly with PW1 protein by 98%.
**Figure 3** shows quantification of flow cytometric analyses of single cells from the hematopoietic system from the PW1 reporter mouse stained with antibodies against Lin, CD34, cKit, and Sca. These markers are used to distinguish the mouse long-term (LT-HSC) and short-term (ST-HSC) hematopoietic stem cells (self-renew-capable), and the Multipotent progenitors (MPP) as well as the Common Lymphoid Progenitor (CLP) that have low or no self-renew capability — the later the developmental stage of MPP, the lesser the self-renewal ability. Quantification shows that the fraction representing LT-HSC and ST-HST is highly enriched in PW1 (ß-galactosidase positive cells) whereas the low self renew fraction is poor in PW1positive cells.

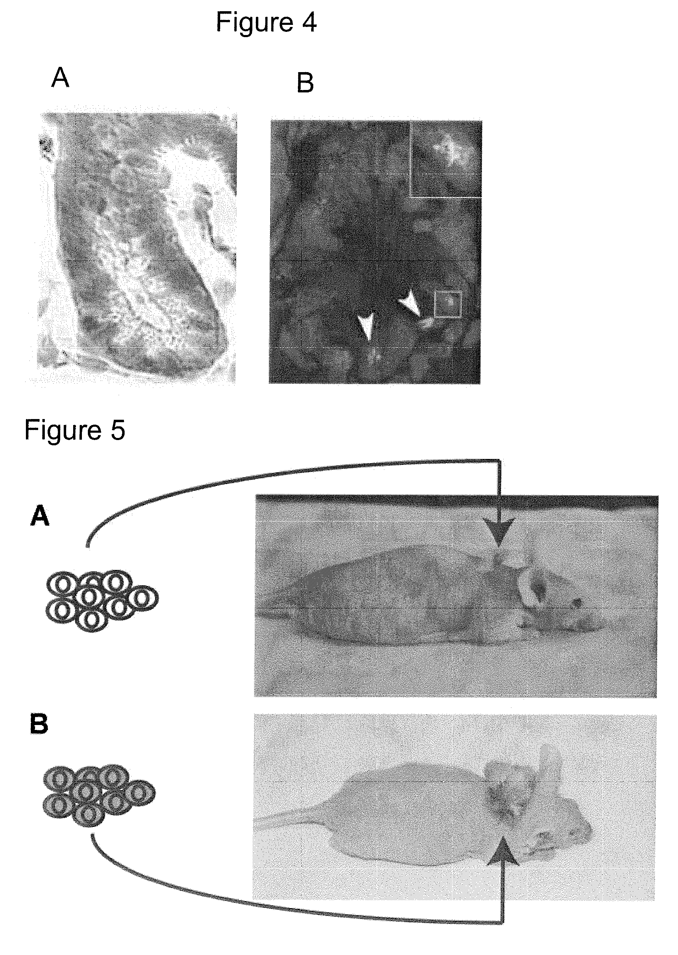

**Figure 4A** shows intestine sections reacted with X-gal. Reporter activity is detected in cells located in the crypt and labeled cells are interspersed among unlabelled cells. This distribution is reminiscent to the cycling crypt stem cells.
**Figure 4B** shows intestine sections immunostained with antibodies against ß-galactosidase (green) and H3P (red) a marker of cycling cells. Some ß-gal+ cells express the G2-M-phase marker phospho-histone H3 confirming reporter activity in the cycling crypt stem cells of the intestinal basal crypt.
**Figures 5A and 5B** show graft experiments on NUDE mice after FAC sorting.
   Figure 5A shows the resulting hair growth after grafting 450 000 ß-gal negative cells from 3 independent FACS experiments.
   Figure 5B shows the resulting hair growth after grafting 450 000 ß-gal positive cells from 3 independent FACS experiments. Only ß-gal positive cells allow an effective hair growth.

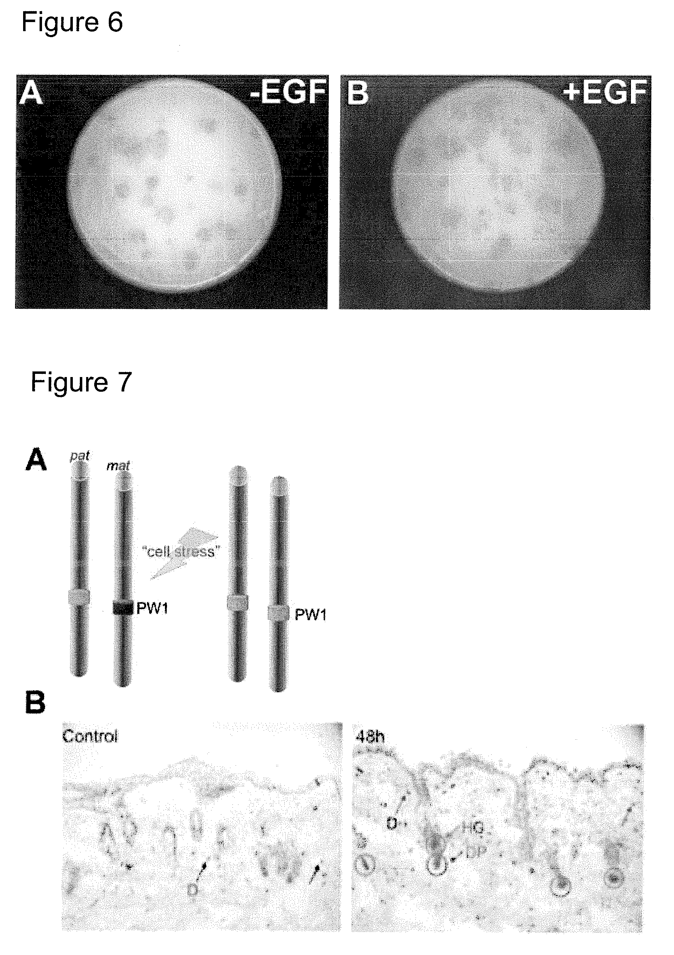

**Figures 6A and 6B** show *in vitro* culture of ß-gal positive cells after FAC sorting in absence (6A) or in presence (6B) of EGF. The results show that in a medium containing EGF, the number of blue clones and the number of blue cells per clone is greater.
**Figure 7A** is a schematic drawing of the cell stress impact induced by a mechanical depilation of the transgenic mice carrying the maternal allele of *Tg(Pw1^{IRES-nLacZ})*.
**Figure 7B** shows photographs of skin sections after LacZ coloration. The figure shows no detection of ß-galactosidase in normal skin sections. However, when a mechanical depilation is performed (injury), the maternal reporter expression is activated in the stem cell niches revealing that stem cell mobilization involves epigenetic changes.

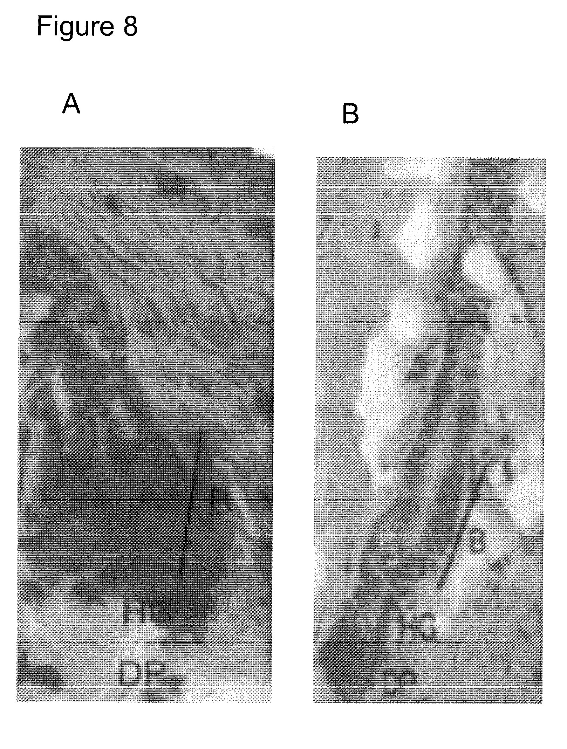

**Figures 8A and 8B** shows photographs of the hair follicle sections in 6-month (Figure 8A) and 11-month (Figure 8B) old transgenic mouse.

With aging, reporter activity is no longer expressed in the bulge but remains unchanged in the dermal papilla compartment meaning that *Pw1* is differentially regulated in these two compartments.

### Detailed description of the invention

### The transgenic non-human animal:

The term "non-human animal" includes any animal, more preferably a vertebrate, still more preferably a mammal, including rodents, sheep, dogs, cats, horses, pigs, cattle, goats, or a primate, as well as a bird. More preferably it is a rodent, such as a mouse, a rat, a guinea pig, a rabbit, and the like. Still more preferably it is a mouse.

The invention makes use of a transgenic non-human animal that expresses a reporter gene. The expression of the reporter gene is controlled by the *Pw1* endogenous promoter which also controls the expression of *Pw1.* As a result, the cells that express PW1 can be identified, through detection of the reporter gene expression. The transgenic non-human animal may have been genetically modified itself, or may be a progeny of a genetically modified non-human animal.

*Pw1*/*Peg3* ("paternally expressed gene 3"), herein designated as *"Pw1",* is a maternally imprinted gene that is expressed primarily during embryogenesis and in adult ovary, testis, muscle, and brain in mouse. In the present invention, the term *"Pw1 or "Peg3*" means the mouse *Pw1* gene or the orthologous gene in any other animal species.

Mammalian imprinting regulates growth and the establishment of parental nurturing behaviors, but the detailed molecular mechanisms by which this occurs are incompletely known. *Pw1* mediates cell stress and pro-survival pathways *in vitro,* as well as muscle atrophy and stem cell *number in vivo.* Kim et al. (2000) mapped the mouse *Pw1*/*Peg3* gene to proximal chromosome 7 and determined that the gene contains 13 exons, the last 4 of which originated from the ancestral ZIM2 gene (Kim et al., 2000). The initiation codon is located in exon 3. Because imprinting is generally conserved among mammals, and imprinted domains generally encompass several adjacent genes, expression patterns and chromosomal environment of the human counterpart of *Peg3* was of interest. Kim et al. (1997) localized the human *PW1*/*PEG3* gene approximately 2 Mb proximal to the telomere of 19q, within a region known to carry large numbers of tandemly clustered Kruppel-type zinc finger-containing (ZNF) genes (Kim et al., 1997).

The transgenic non-human animal is modified with a reporter gene that is operatively linked to *Pw1*. The transgenic non-human animal may be produced by various strategies. In a particular embodiment, the transgenic non-human animal may be generated or may be a progeny of a non-human animal generated by introduction, into a non-human animal ovocyte, of a BAC recombinant vector comprising a reporter gene inserted into a *Pw1* gene. Details on the generation of a transgenic mouse useful in the invention, called *Tg(Pw1*^{*IRES-nLacZ*/}*⁺)* mouse, are provided in Example 1.

The reporter gene may be any gene that is detectable by a chromogenic, luminescent, or fluorescent signal. Examples of reporter genes encode enzymes such as β-lactamase, β-galactosidase, alkaline phosphatase, SEAP (secreted alkaline phosphatase). Examples of fluorescent agents include green, red or yellow fluorescent proteins. Examples of luminescent agents include luciferase proteins (such as firefly, renilla, gaussia luciferase).

In a preferred embodiment, the reporter gene is the LacZ gene that encodes the β-galactosidase enzyme. β-galactosidase cleaves the colorless substrate X-gal (5-bromo-4-chloro-3-indolyl-β-galactopyranoside) into galactose and a blue insoluble product. In another preferred embodiment, the reporter gene encodes the Green Fluorescent Protein (GFP).

Antibodies against the protein product of the reporter gene may be used to immunostain the cells that express the reporter gene. The antibodies may be detectably labeled, or may be revealed by indirect labeling.

### Isolation and culture of adult stem cells:

The non-human animal model used in the present invention provides a single step setting in which stem cells can be immediately recognized in their normal tissue context or in response to experimental manipulation. This non-human animal can be used to identify the quiescent and/or proliferative stem cells and their niche in all adult tissues. These include e.g. blood, bone marrow, hematopoietic system, skin, hair follicle, muscle, nervous system, heart, intestine, thymus, pancreas, testis, eye, kidney, liver, lung, spleen, tongue, bones and dental pulp.

It is herein provided a method for cultivating non-human animal adult stem cells, which method comprises the steps consisting of:
a. Providing a transgenic non-human animal expressing a reporter gene detectable by a chromogenic, luminescent or fluorescent signal which identifies the cells that express *Pw1* that is a marker of adult stem cells;
b. Isolating adult stem cells from the transgenic non-human animal by analyzing the chromogenic, luminescent or fluorescent signal of the reporter gene, optionally coupled to an analysis of stem cell marker(s);
c. Allowing the isolated adult stem cells to grow *in vitro,* preferably during at least 5, preferably at least 8, preferably at least 10, preferably at least 12 passages.

The chromogenic, luminescent or fluorescent signal of the reporter gene is still detectable after at least 12 passages.

In a particular embodiment, the adult stem cells can be purified up to about 95 to about 98% purity using FACs analyses coupled with detection of the reporter gene.

More particularly, clonogenicity assay *in vitro* and graft experiments *in vivo* show that *Pw1* participates in the hair follicle regeneration and the PW1+ cells (which appear in blue when the *Pw1* promoter is activated) act as hair follicle stem cells. Moreover, these purified blue stem cells can be cultured *in vitro* while keeping their "stemness" and their capacity of *regeneration in vivo.*

### Screening methods

The transgenic non-human animal expressing a reporter gene detectable by a chromogenic, luminescent or fluorescent signal which identifies the cells that express PW1, or PW1-expressing cells or tissues isolated therefrom, are useful for screening or identifying a candidate substance for its ability to stimulate adult stem cells.

The candidate substance may be any substance of defined or undefined structure, including a chemical drug, a biological compound, e.g. antibodies, nucleic acids or, peptides, or a mixture of natural compounds, e.g. an extract of a plant. In a preferred embodiment, it is a pharmaceutical drug, *i.e.* a drug that is pharmaceutically acceptable.

The candidate substance that is hereby selected is of interest in tissue repair, which may be useful in treating neurodegenerative diseases, including stroke and Alzheimer's disease, in spinal cord injury, as well as cardiovascular diseases, in particular myocardial infarction. Another field of regenerative medicine is skin repair, in particular for bums or genetic diseases).

The screening of candidate substances may be performed *in vitro* or *in vivo.* According to the invention, candidate substances are evaluated for their ability to trigger or maintain the signal from the reporter gene.

More particulary, the invention provides *an in vitro* method for screening a candidate substance for its ability to stimulate adult stem cells, which method comprises the steps consisting of:
a. Providing adult stem cells isolated from a transgenic non-human animal expressing a reporter gene detectable by a chromogenic, luminescent, or fluorescent signal, which identifies the cells that express PW1, that is a marker of adult stem cells;
b. Contacting said stem cells with the candidate substance;
c. Determining the ability of the candidate substance to trigger, maintain or enhance the chromogenic, luminescent or fluorescent signal, whereby identifying the substance as being able to stimulate adult stem cells.

In a particular embodiment, the stem cells of step (a) are isolated by an analysis of the chromogenic, luminescent or fluorescent signal of the reporter gene, optionally coupled to an analysis of stem cell marker(s), as *Sca, cKit, CD34,* and wherein step (c) comprises determining the ability of the candidate substance to enhance the signal.

In another particular embodiment, the stem cells of step (a) are isolated by an analysis of stem cell marker(s) without any chromogenic, luminescent or fluorescent signal being detectable, and wherein step (c) comprises determining the ability of the candidate substance to trigger said signal.

More particularly the stem cells of step (a) may be isolated from a transgenic non-human animal that has been subjected to a stress, consisting preferably of hypoxia, NO-induced stress, H2O2-induced stress, thermal stress (e.g. at about 42°C), or a chemically induced stress, e.g. with a histone inhibitor. Such stress impacts cell stem function and PW1 expression.

Stress can activate expression of the reporter gene regardless of the imprinting. In that context, the above method allows for screening a candidate substance for its ability to induce expression of the reporter gene that is maternally transmitted and silenced in the non-human transgenic animal.

The stem cells of step (a) may be isolated from any tissue such as blood, bone marrow, hematopoietic system, skin, hair follicle, muscle, nervous system, heart, intestine, thymus, pancreas, testis, eye, kidney, liver, lung, spleen, tongue, bones and, dental pulp.

When the reporter gene has been introduced through a BAC recombination, such as herein described with respect with the *Tg(Pw1^{IRES-nLacZ})* mouse of Example 1, the reporter gene is parentally imprinted (paternally expressed). The maternal repression of the *Pw1* allele is lost following stem cell mobilization in a few tissues thus far examined. Adult stem cells from the non-human animals with the maternal BAC (white cells) can then be isolated, and then used to screen small molecules that have the capacity to switch on the cells (e.g. to turn them blue when LacZ is used as the reporter gene, and X-gal is used to reveal the activity of this reporter gene), in other words the molecules that allow to activate or to mobilize these stem cells. Thus, it is possible to use this epigenetic control as readout for any candidate substance such as pharmaceutical drugs that can affect mobilization.

The invention further provides *an in vivo* method for screening a candidate substance for its ability to stimulate adult stem cells, which method comprises administering a transgenic non-human animal expressing a reporter gene detectable by a chromogenic, luminescent or fluorescent signal which identifies the cells that express PW1, that is a marker of adult stem cells, with the candidate substance; and determining the ability of the candidate substance to trigger, maintain or enhance the chromogenic, luminescent or fluorescent signal, whereby identifying the substance as being able to stimulate adult stem cells.

Before, during, or sequentially with, administration of the candidate substance, the non-human animal may be subjected to a stress which impacts cell stem function, and PW1 expression.

A candidate substance is then selected for its ability to revert the maternal allele expression, *i.e.* for its ability to induce the reporter gene expression in said transgenic non-human animal carrying the silenced maternally transmitted transgene reporter.

### Marker of cell aging

The transgenic non-human animal used in the present invention shows changes in expression of the reporter gene as a function of age, which is of paramount interest for research about aging and regenerative medicine. More particularly the inventors found that reporter activity is constitutive in the stem cells of skin up to about 6 months of age, whereby by more than about 11months of age, reporter activity is lost in the bulge cells although the stem cells are still present but remains unchanged in the dermal papilla. Other experiments revealed that unless PW1 is 'reactivated' prior to using the old stem cells for engraftment, regeneration is very poor. Accordingly the transgenic mice or PW1-expressing cells or tissues isolated therefrom, are useful for monitoring cell aging, in particular aging of adult stem cells present in the hair follicle. Moreover, PW1 reporter activity is an important new tool also for assessing stem cell regenerative capacity.

The term "monitoring cell aging" includes studying changes in stem cell function as a model for human aging, in particular human skin aging.

The Examples and Figures illustrate the invention without limiting its scope.

### Examples

### Example 1: Generation of Tg(Pw1^{IRES-nLacZ}) mice

Transgenic mice have been generating using BAC recombining strategy, as previously described (Lee et al., 2001). The *Pw1*-containing BAC clone (ID# 508P6, 180 kb) comes from 129Sv library. The BAC contains the 26,5 kb corresponding to *Pw1* gene and the BAC contains at least 80 kb of sequence in the 5' and 3' part of *Pw1*. The BAC contains also 34 kb downstream of Pw1, *zim1.* A kanamycine cassette surrounded by FRT sites was introduced into an IRESnLacZpA containing plasmid (3,8 kb) (Relaix 2004). The resulting cassette was subcloned into the XbaI site of pBSK plasmid containing a 485 bp homologous sequence (5' of exon 9 of genomic *pw1,* location: + 19302 bp of Pw1 genomic sequence, accession no. ENSMUSG00000002265; NCBIM37). The cassette was introduced in bacterial cells containing the BAC by electroporation. Selected colonies (Kanamycin positive cells) containing the targeting construct were submitted to arabinose, leading to the excision of the kanamycin cassette. Finally, the *Tg(Pw1^{IRES-nLacZ})* BAC was injected into ovocytes to generate founders. Germline-transmitted allele was identified by PCR (primer_Ex9: 5'-CCACATTCCTTACACTCTAAAGC-3' (SEQ ID NO:1) and primer_dLacZ: 5'-CCGCTACAGTCAACAGCAAC-3' (SEQ ID NO:2)).

The *Tg(Pw1^{IRES-nLacZ})* reporter mice are maintained in a C57BL6/J background.

### Example 2: Identification of adult stem cells expressing PW1 marker

### 2.1. Materials and methods

### X-Gal staining, whole-mount in situ hybridization, histology, immunohistochemistry

Mouse embryos were collected after natural overnight matings. Dissected embryos were fixed and stained with X-Gal (Roche) as described previously (Relaix et al., 2004). Whole *mount in situ* hybridization with digoxigenin-labeled riboprobes was performed as described (Tajbakhsh et al., 1997). The Pw1 riboprobe (865 pb) was generated from the PstI digestion of the exon 9 cDNA.

Tissues were fixed with 4% paraformaldehyde (PFA, w/v) in PBS at 4°C and placed in 15% PBS-sucrose overnight at 4°C before being embedded in OCT™(Tissue-Tek® O.C.T), except for the tibialis anterior muscles which were snap frozen in liquid nitrogen-cooled isopentane without previous fixation. Cryosections (8 µm) and cytospin preparations were fixed 15 min at room temperature with 4% PFA (paraformaldehyde) before X-Gal staining (Relaix et al., 2004) for 3-5 hours or processed for immunofluorescence as previously described (Coletti et al., 2002; Nicolas et al., 2005; Schwarzkopf et al., 2006). Primary antibodies: PW1 (Relaix et al., 1996), b-Gal (Promega), laminin (Sigma), M-cadherin (NanoTools), Vimentin (Santacruz), Keratin15 (K15, BD Biosciences), CD34-biotin (eBiosciences), CD49f (BD Biosciences), GFAP (Glial Fibrillary Acidic Protein, DakoCytomation), DCX (Doublecortin, Santa Cruz Biotechnology), EGF-R (EGF Receptor, Upstate Biotechnology), phospho-histone H3 (Upstate Biotechnology). Antibody binding was revealed using species-specific secondary antibodies coupled to Alexa Fluor 488 (Molecular Probes), FITC (DakoCytomation), Cy3 or Cy5 (Jackson Immunoresearch). Nuclei were counterstained with DAPI (Sigma) or nuclear fast red (Sigma). For quantitative analyses of immunostained tissue, positive cells in at least 350 fibers from randomly chosen fields were counted from 3 animals.

### FACS analysis

For fluorescence-activated cell sorting, dorsal skin from 7 week-old T*g(Pw1*^{*IRES-nLacZ*/}*⁺)* mice (n = 3-5) were collected and cell preparation was obtained as previously described (Jacks et al, 2008). Cells were stained with 10 ng/ml of the following primary antibodies: rat anti-mouse CD34-Pacific blue (Clinisciences) and rat anti-mouse CD49f-PE (BD Biosciences). Hematopoietic cells were flushed from the bone marrow of 7 week-old mice (n=10) with PBS containing 1% BSA. Cells were filtrated and treated with 154 mM NH4C1, 10 mM KHCO3 and 0,1 mM EDTA to eliminate red blood cells. Cells were incubated 1 hour at 4°C with 10 ng/ml of the following antibodies: rat anti-mouse hematopoietic lineage flow cocktail-Pacific blue (Lin: CD3, CD45R/B220, CD11b, TER-119, Ly-6G), rat anti-mouse CD34-biotin (Ram34), rat anti-mouse Scal-PE, rat anti-mouse cKit-APC (all from BD Biosciences). Cell pellets were washed prior to incubation with Streptavidin-PE-Cy7 (BD Biosciences) for 30 min on ice. For detection of nuclear β-galactosidase activity, a fluorescein di-β-D-galactopyranoside (FDG) staining kit (Invitrogen) was used according to manufacturer's instructions. Staining analysis was carried out using a FACSAria (Becton Dickinson). ß-galactosidase positive cells were defined as having a signal superior to the signal from the cells isolated from non transgenic mouse.

### Microscopy and live cell imaging

Images were acquired using a Leica DM fluorescence microscope or Leica SPE confocal microscope.

### 2.2. Results

The reporter expression profile of mouse embryo was detected using X-gal staining (see **Figure 1B****).**

This mouse model identified quiescent or proliferative stem cell niches in adult tissues examined: muscle (See **Figures 2A and 2B****)**, cells from the hematopoietic system (see **Figure 3****),** intestine (**see** **Figures 4A, and 4B****)**, hair follicle, central nervous system, epicardium, bone and, testis,

Using *Tg(Pwl*^{*IRES-nLacz*/}*⁺)* mice, the inventors have isolated skin stem cells to 98% purity using FACs analyses coupled with a fluorescent substrate for ß-galactosidase. The purified cells are long-lived and can be maintained in culture for several passages. Clonogenicity *assays in vitro* and graft experiments *in vivo* show that PW1 participates in hair follicle regeneration and the PW1+ cells (blue cells) display all the hallmarks of hair follicle stem cells. These results indicate that these purified blue stem cells can be *cultured in vitro* while keeping their stemness and thus their capacity of regeneration *in vivo.* (see Figures 5A and 5B).

### Example 3: Screening methods

### 3.1. In vitro testing the capacity of adult stem cell regeneration

The inventors isolated cells from a transgenic mouse as obtained in Example 1, and isolated them by FAC sorting. Only the blue cells were *cultured in vitro.*

Then for proof of concept, the cells were contacted with human recombinant EGF or without EGF. The growth factor was tested for its ability to keep the cells blue over time. The results show that in a medium containing EGF, the number of blue clones as well as the number of blue cells per clone was higher (see **Figures 6A and 6B****)**.

### 3.2. In vivo testing the capacity of adult stem cell regeneration

In mice carrying the maternal allele of *Tg(Pw1^{IRES-nLacZ})* reporter expression is detected in few cells located in the dermis (D) but no expression is detected in the bulge nor in the dermal papilla.

For proof of concept, the mice carrying the maternal allele of *Tg(Pw1^{IRES-nLacZ})* were injured by mechanical stress (here depilation). The inventors show that 48 hours after injury reporter expression is turned on, which is concomitant with the regeneration cycle of the hair follicle (Figures 7A and 7B). After the regeneration cycle, reporter activity is turned off.

### Example 4: Mobilization of old adult stem cells using Tg(Pw1^{IRES-nLacZ/}⁺) mice

### 4.1. Material methods: graft experiments

Pieces of back skin (whole skin dermis+epidermis) were removed from reporter mice and lied on the back of athymic (NUDE) mice where the same size of back skin has been previously removed. The graft was sutured and a tulle gras dressing was applied to prevent dessication. The graft was protected with a bandage for one week. The hair healing was visible 3 weeks after grafting. The whole graft was stained with X-Galactosidase and then embedded in OCT. 10 µm sections of the graft were performed and stained again with X-Galactosidase.

### 4.2. Results

The activity of the reporter is constitutive in the stem cells of skin up to ∼6 months of age, whereby by >11months of age, reporter activity is lost in the bulge cells although the stem cells are still present but remains unchanged in the dermal papilla **(****Figures 8A and 8B****)**. The inventors grafted to NUDE mice pieces of skin from young (6 months old) or old (>11 months) *Tg(Pw1*^{*IRES-nLacZ*/}*⁺)* mice. The results show that regeneration capacity of old stem cells is very poor.

A second experiment in which the old *Tg(Pw1^{IRES-nLacZl+})* mice were injured by mechanical stress (here depilation) show that the activity of the reporter was turned on. The inventors grafted pieces of skin from the injured old mice into NUDE mice. The results show that the regeneration capacity of the β-galactosidase + old skin is comparable to the regeneration capacity of young skin.

### References

Barker, N., Huch, M., Kujala, P., van de Wetering, M., Snippert, H.J., van Es, J.H., Sato, T., Stange, D.E., Begthel, H., van den Born, M., et al. (2010). Lgr5(+ve) stem cells drive self-renewal in the stomach and build long-lived gastric units in vitro. Cell Stem Cell 6, 25-36.
Blanpain, C., and Fuchs, E. (2006). Epidermal stem cells of the skin. Annu Rev Cell Dev Biol 22, 339-373.
Coletti, D., Yang, E., Marazzi, G., and Sassoon, D. (2002). TNFalpha inhibits skeletal myogenesis through a PW1-dependent pathway by recruitment of caspase pathways. EMBO J 21, 631-642.
Jaks, V., Barker, N., Kasper, M., van Es, J. H., Snippert, H. J., Clevers, H., Toftgard, R. (2008). Lgr5 marks cycling, yet long-lived, hair follicle stem cells. Nat Genet 40, 1291-9.
Kim, J., Ashworth, L., Branscomb, E., and Stubbs, L. (1997). The human homolog of a mouse-imprinted gene, Peg3, maps to a zinc finger gene-rich region of human chromosome 19q13.4. Genome Res 7, 532-540.
Kim, J., Bergmann, A., and Stubbs, L. (2000). Exon sharing of a novel human zinc-finger gene, ZIM2, and paternally expressed gene 3 (PEG3). Genomics 64, 114-118.
Lee, E.C., Yu, D., Martinez de Velasco, J., Tessarollo, L., Swing, D.A., Court, D.L., Jenkins, N.A., and Copeland, N.G. (2001). A highly efficient Escherichia coli-based chromosome engineering system adapted for recombinogenic targeting and subcloning of BAC DNA. Genomics 73, 56-65.
Mitchell, K.J., Pannerec, A., Cadot, B., Parlakian, A., Besson, V., Gomes, E.R., Marazzi, G., and Sassoon, D.A. (2010). Identification and characterization of a non-satellite cell muscle resident progenitor during postnatal development. Nat Cell Biol 12, 257-266. Moore, K.A., and Lemischka, I.R. (2006). Stem cells and their niches. Science 311, 1880-1885.
Morrison, S.J., Shah, N.M., and Anderson, D.J. (1997). Regulatory mechanisms in stem cell biology. Cell 88, 287-298.
Nicolas, N., Marazzi, G., Kelley, K., and Sassoon, D. (2005). Embryonic deregulation of muscle stress signaling pathways leads to altered postnatal stem cell behavior and a failure in postnatal muscle growth. Dev Biol 281, 171-183.
Relaix, F., Rocancourt, D., Mansouri, A., and Buckingham, M. (2004). Divergent functions of murine Pax3 and Pax7 in limb muscle development. Genes Dev 18, 1088-1105.
Relaix, F., Weng, X., Marazzi, G., Yang, E., Copeland, N., Jenkins, N., Spence, S.E., and Sassoon, D. (1996). Pw1, a novel zinc finger gene implicated in the myogenic and neuronal lineages. Dev Biol 177, 383-396.
Schwarzkopf, M., Coletti, D., Sassoon, D., and Marazzi, G. (2006). Muscle cachexia is regulated by a p53-PW1/Peg3-dependent pathway. Genes Dev 20, 3440-3452.
Tajbakhsh, S., Rocancourt, D., Cossu, G., and Buckingham, M. (1997). Redefining the genetic hierarchies controlling skeletal myogenesis: Pax-3 and Myf-5 act upstream of MyoD. Cell 89, 127-138.

## Claims

1. *An in vitro* method for screening a candidate substance for its ability to stimulate adult stem cells, which method comprises the steps consisting of:
a. Providing adult stem cells isolated from a transgenic non-human animal expressing a reporter gene detectable by a chromogenic, luminescent, or fluorescent signal which identifies the cells that express Pw1 that is a marker of adult stem cells;
b. Contacting said stem cells with the candidate substance;
c. Determining the ability of the candidate substance to trigger, maintain or enhance the chromogenic, luminescent or fluorescent signal, whereby identifying the substance as being able to stimulate adult stem cells.

2. The method of claim 1, wherein the stem cells of step (a) are isolated by an analysis of the chromogenic, luminescent or fluorescent signal of the reporter gene, optionally coupled to an analysis of stem cell marker(s), and wherein step (c) comprises determining the ability of the candidate substance to enhance the signal.

3. The method of claim 1, wherein the stem cells of step (a) are isolated by an analysis of stem cell marker(s) without any chromogenic, luminescent or fluorescent signal being detectable, and wherein step (c) comprises determining the ability of the candidate substance to trigger said signal.

4. The method of claim 3, wherein the stem cells of step (a) are isolated from a transgenic non-human animal that has been subjected to a stress, consisting preferably of hypoxia, NO-induced stress, H₂O₂-induced stress, thermal stress, or a chemically induced stress, e.g. with a histone inhibitor.

5. The method of claim 4, wherein the expression of the reporter gene is maternally transmitted and silenced in the non-human transgenic animal, and the candidate substance is screened for its ability to induce expression of said reporter gene.

6. The method according to any of claims 1 to 5, wherein the stem cells of step (a) are isolated from a tissue selected from the group consisting of blood, bone marrow, hematopoietic system, skin, hair follicle, muscle, nervous system, heart, intestine, thymus, pancreas, testis, eye, kidney, liver, lung, spleen, tongue, bones and dental pulp.

7. *An in vivo* method for screening a candidate substance for its ability to stimulate adult stem cells, which method comprises administering a transgenic non-human animal expressing a reporter gene detectable by a chromogenic, luminescent or fluorescent signal which identifies the cells that express Pw1, that is a marker of adult stem cells, with the candidate substance; and determining the ability of the candidate substance to trigger, maintain or enhance the chromogenic, luminescent or fluorescent signal, whereby identifying the substance as being able to stimulate adult stem cells.

8. A method for cultivating non-human animal adult stem cells, which method comprises the steps consisting of:
a. Providing a transgenic non-human animal expressing a reporter gene detectable by a chromogenic, luminescent or fluorescent signal which identifies the cells that express Pw1 that is a marker of adult stem cells;
b. Isolating adult stem cells from the transgenic non-human animal by analyzing the chromogenic, luminescent or fluorescent signal of the reporter gene, optionally coupled to an analysis of stem cell marker(s);
c. Allowing the isolated adult stem cells to *grow in vitro.*

9. The method according to any of claims 1 to 8, wherein the transgenic non-human animal has been generated or is a progeny of a non-human animal generated by introduction, into a non-human animal ovocyte, of a BAC recombinant vector comprising a reporter gene inserted into a PW1 gene.

10. The method according to any of claims 1 to 9, wherein the reporter gene encodes β-galactosidase or Green Fluorescent Protein.

11. Use of a transgenic non-human animal expressing a reporter gene detectable by a chromogenic, luminescent or fluorescent signal which identifies the cells that express Pw1, or of Pw1-expressing cells or tissues isolated therefrom, as a model for screening a candidate substance for its ability to stimulate adult stem cells.

12. The use of claim 11, wherein the candidate substance is a chemical drug, a biological compound, or a mixture of natural compounds.

13. Use of a transgenic non-human animal expressing a reporter gene detectable by a chromogenic, luminescent or fluorescent signal which identifies the cells that express Pw1, or of Pw1-expressing cells or tissues isolated therefrom, as a model for monitoring cell aging.

14. The use of claim 13, for monitoring aging of adult stem cells present in skin or hair follicle.

15. The method of any of claims 1 to 10, or the use of any of claims 11 to 14, wherein the non-human animal is a mammal, preferably a rodent, more preferably a mouse.


SEQUENCE LISTING

<110>  Université Pierre et Marie Curie (Paris 6)

<120>  A transgenic animal as a model for identifying adult stem cells,
       and uses thereof

<130>  B1054

<160>  2

<170>  PatentIn version 3.3

<210>  1
<211>  23
<212>  DNA
<213>  Artificial

<220>
<223>  Primer

<400>  1
ccacattcct tacactctaa agc                                              23


<210>  2
<211>  20
<212>  DNA
<213>  Artificial

<220>
<223>  primer

<400>  2
ccgctacagt caacagcaac                                                  20